# EUROPEAN PATENT APPLICATION

(11) **EP 4 559 515 A1**
(43) Date of publication of application: **28.05.2025**
(21) Application number: 22952052.3
(22) Date of filing: 27.07.2022
(51) Int. Cl.: A61N 1/39, A61H 31/00, A61B 5/346, A61B 5/00

(54) **EMERGENCY TREATMENT DEVICE FOR CHEST COMPRESSION AND DEFIBRILLATION OF PATIENT AND METHOD FOR CORRECTING LOSS OF CHEST COMPRESSION DEPTH OF PATIENT**

(30) Priority: 20.07.2022 KR 20220089795
(71) Applicant: CU Medical Systems Inc., Wonju-si, Gangwon-do 26365 (KR)
(72) Inventor: CHOI, Yeong-ho, Seoul 03440 (KR); SONG, In-ho, Seoul 02799 (KR)
(74) Representative: Schiweck Weinzierl Koch Patentanwälte Partnerschaft mbB
(86) International application number: PCT/KR2022/011006
(87) International publication number: WO 2024/019203

(57) **Abstract**

According to one embodiment of the present invention, a method for correcting for loss of a chest compression depth of a patient includes a) setting a value of a chest compression start position of the patient to start chest compression of the patient with a lower end of a piston positioned above a chest of the patient, and then expanding or contracting the piston so that the lower end of the piston moves to the chest compression start position according to the value of the chest compression start position of the patient, by a control unit, b) applying a value of an initial chest compression depth which is a movement length value of the piston from the chest compression start position of the patient toward an upper side of a base plate supporting a back of the patient and a threshold value of the chest compression depth to expand and contract the piston so that chest compression is performed, and detecting a change in a shape of the chest of the patient during chest compression to update the chest compression start position of the patient, by the control unit, c) comparing and determining a value obtained by adding a separation distance between a cap fastened to the lower end of the piston and the chest of the patient and the value of the initial chest compression depth and the threshold value of the chest compression depth, by the control unit, and d) expanding and contracting the piston so that the cap reciprocates according to the value of the initial or updated chest compression depth to perform the chest compression by the cap.

## Description

### [Technical Field]

The present invention relates to an emergency treatment device for chest compression and defibrillation of a patient and a method for correcting for loss of a chest compression depth of a patient. More specifically, the present invention relates to an emergency treatment device for chest compression and defibrillation of a patient and a method for correcting for loss of a chest compression depth of a patient due to chest shape deformation that occurs in a process of compressing a chest of a patient with a cap provided in the emergency treatment device, thereby ensuring that the chest of the patient is compressed accurately.

### [Background Art]

Cardiopulmonary resuscitation (CPR) is a technique that involves a series of repeated procedures, including chest compressions, airway maintenance, artificial respiration, and the like. More specifically, when a suspected patient with cardiac arrest occurs, the cardiopulmonary resuscitation is performed by checking the safety of the scene, checking the response and breathing state of the fallen person, asking for help from those around them, quickly reporting it to the emergency rescue agency, and clasping both his/her hands and using the heels of his/her hands to compress the center of the chest (avoiding the pubic area, and the center of both nipples) 30 times with his/her elbows extended, but with a compression depth of less than 4 to 6 cm. Moreover, after the chest compressions, the cardiopulmonary resuscitation is performed by securing the airway and performing artificial respiration twice, once for 1 to 25 seconds, while checking whether the chest rises, and performing the chest compressions and artificial respirations alternately twice.

However, in the case of the general public, even when they have received CPR training, there are problems such as the unfamiliarity of the cardiopulmonary resuscitation in the event of a cardiac arrest, the burden of rib damage, and the inability to accurately determine the depth of compression because the chest height is not consistent for each patient.

Accordingly, various types of cardiopulmonary resuscitation (CPR) devices are known. One of the devices is driven by compressed air or breathing gas (Jolife AB, Lund, Sweden; LucasTM). A unique advantage of the cardiopulmonary resuscitation device is light weight thereof, which makes it portable. Another advantage is the elastic properties of compressed air, which makes gas-driven cardiopulmonary resuscitation devices less likely to cause damage to a chest of a patient than devices with rigid compression means. The known device may be used as an emergency device in life-saving situations. Furthermore, in a known device, the motive gas may be supplied from a hospital air supply line which may be desirable for uninterrupted bursts of cardiopulmonary resuscitation when a patient is admitted.

However, the cardiopulmonary resuscitation device does not have a function to analyze electrocardiogram (ECG), making it difficult to accurately check whether the patient is in cardiac arrest.

As such, since the cardiopulmonary resuscitation devices have the problem of being difficult to accurately check whether a patient is in cardiac arrest, there is a need to implement a device that integrates an automated external defibrillator (AED), a device that analyzes the electrocardiogram in emergency situations, and use the integrated device in emergency situations.

Meanwhile, the device that combines the cardiopulmonary resuscitation device and the automated external defibrillator has been disclosed in Korean Patent No. 10-1956053 (Title of Invention: Cardiopulmonary resuscitation device with adjustable compression depth when chest is compressed, hereinafter referred to as "prior document").

The above-mentioned prior document is a cardiopulmonary resuscitation device including a chest compression unit having a chest compression means for compressing a chest of a patient, a control means for analyzing electrocardiogram of the patient and controlling an electric shock, and a defibrillator unit having pads attached to the patient, in which the lengths of a hydraulic frame and a height-adjusting frame are adjusted so that the chest compression unit is positioned in an upper region toward the chest of the patient, thereby allowing cardiopulmonary resuscitation to be performed without burden by adjusting the chest compression depth of the chest compression unit.

However, the prior document has a problem in that when the chest of the patient is compressed in the initial state of the chest of the patient before the chest is compressed, the chest of the patient is pressed, the shape of the chest is deformed, and the depth of chest compression is lost, resulting in an error in the depth of chest compression. As a result of this error, it is difficult to accurately perform the cardiopulmonary resuscitation using the chest compression unit, and therefore it is difficult to provide the effect of the prior document that the cardiopulmonary resuscitation can be performed without burden.

### [Disclosure]

### [Technical Problem]

Accordingly, an object of the present invention is to provide a method for correcting for loss of a chest compression depth of a patient by correcting for the loss of the chest compression depth due to a change of a chest shape that occurs in a process of compressing a chest of the patient with a cap provided in an emergency treatment device for chest compression and defibrillation of the patient, thereby ensuring that the chest of the patient is compressed accurately.

In addition, an object of the present invention is to provide a method for correcting for loss of a chest compression depth of a patient, which automates the process of chest compression and defibrillation of the patient, performs compression at the existing compression depth when the sum of a set initial chest compression depth and a separation distance is less than a threshold value of a compression depth, and performs chest compression at the corrected compression depth when the sum exceeds the threshold value, thereby improving the accuracy and stability of the process of chest compression and defibrillation of a patient and improving a user's convenience during the chest compression and defibrillation of the patient.

Moreover, an object of the present invention is to provide a method for correcting for loss of a chest compression depth of a patient, which can detect whether there is a change in a chest compression start position periodically, audibly/visually notify a user of a change in the shape of the chest of the patient, and update the chest compression start position.

However, the technical problems to be achieved in the present invention are not limited to the technical problems mentioned above, and other technical problems not mentioned can be clearly understood by a person having ordinary knowledge in the technical field to which the present invention belongs from the description below.

### [Technical Solution]

An emergency treatment device for chest compression and defibrillation of a patient according to one embodiment of the present invention, which is a technical means for achieving the above-mentioned objects, may include: a base plate configured to support a back of the patient; a support having one end and the other end coupled to both edges of the base plate; a hood which coupled to one side of the support and positioned above a chest of the patient and to which a piston for compressing the chest of the patient is connected; and a control unit which sets a value of a chest compression start position of the patient to start chest compression of the patient with a lower end of the piston, and then, expands or contracts the piston so that the lower end of the piston moves to the chest compression start position according to the value of the chest compression start position, applies a value of an initial chest compression depth which is a movement length value of the piston from the chest compression start position toward an upper side of the base plate and a threshold value of the chest compression depth to expand or contract the piston so that the chest compression is performed, detects a change in a shape of the chest of the patient during the chest compression to update the chest compression start position of the patient, compares and determines a value obtained by adding a separation distance between the cap fastened to the lower end of the piston and the chest of the patient and the value of the initial chest compression depth, and the threshold value of the chest compression depth, and expands and contracts the piston so that the cap reciprocates according to the value of the initial or updated chest compression depth to perform the chest compression by the cap.

A method for correcting for loss of a chest compression depth of a patient according to an embodiment of the present invention, which is performed in an emergency treatment device for chest compression and defibrillation of a patient, may include: a) setting a value of a chest compression start position of the patient to start chest compression of the patient with a lower end of a piston positioned above a chest of the patient, and then expanding or contracting the piston so that the lower end of the piston moves to the chest compression start position according to the value of the chest compression start position of the patient, by a control unit; b) applying a value of an initial chest compression depth which is a movement length value of the piston from the chest compression start position of the patient toward an upper side of a base plate supporting a back of the patient and a threshold value of the chest compression depth to expand and contract the piston so that chest compression is performed, and detecting a change in a shape in the chest of the patient during chest compression to update the chest compression start position of the patient, by the control unit; c) comparing and determining a value obtained by adding a separation distance between a cap fastened to the lower end of the piston and the chest of the patient and the value of the initial chest compression depth, and the threshold value of the chest compression depth, by the control unit; and d) expanding and contracting the piston so that the cap reciprocates according to the value of the initial or updated chest compression depth to perform the chest compression by the cap.

### [Advantageous Effects]

The present invention has the effect of correcting for the loss of the chest compression depth due to the change in the chest shape that occurs in the process of compressing the chest of the patient with the cap provided in the emergency treatment device for the chest compression and defibrillation of the patient, thereby ensuring that the chest of the patient is compressed accurately.

In addition, according to the present invention, it is possible to automate the process of the chest compression and defibrillation of the patient, perform the compression at the existing compression depth when the sum of the set initial chest compression depth and the separation distance is less than the threshold value of a compression depth, and perform the chest compression at the corrected compression depth when the sum exceeds the threshold value, thereby improving the accuracy and stability of the process of the chest compression and defibrillation of the patient and improving a user's convenience during the chest compression and defibrillation of the patient.

In addition, according to the present invention, it is possible to detect whether there is a change in the chest compression start position periodically, audibly/visually notify a user of the change in the shape in the chest of the patient, and update the chest compression start position to secure the user's convenience.

However, the effects obtainable from the present invention are not limited to the effects mentioned above, and other effects not mentioned will be clearly understood by those skilled in the art to which the present invention belongs from the description below.

### [Description of Drawings]

FIG. 1 is a perspective view illustrating a partial configuration of an emergency treatment device for chest compression and defibrillation of a patient according to one embodiment of the present invention.
FIG. 2 is a schematic diagram illustrating the partial configuration of the emergency treatment device for the chest compression and defibrillation of the patient illustrated in FIG. 1.
FIG. 3 is a block diagram illustrating a unit equipped in a hood according to one embodiment of the present invention.
FIG. 4 is a diagram illustrating a storage unit and stored information provided in an emergency treatment device for chest compression and defibrillation of a patient according to one embodiment of the present invention.
FIG. 5 is a diagram illustrating a sensor equipped in the emergency treatment device for chest compression and defibrillation of the patient according to one embodiment of the present invention.
FIG. 6 is a diagram illustrating a notification unit [81] equipped in an emergency treatment device for chest compression and defibrillation of a patient according to one embodiment of the present invention.
FIG. 7 is a diagram illustrating a symmetric profile according to one embodiment of the present invention.
FIG. 8 is a perspective view of a cap according to one embodiment of the present invention.
FIG. 9 is a cross-sectional view taken along line A-A of FIG. 8.
FIG. 10 is a perspective view illustrating a first member according to one embodiment of the present invention.
FIG. 11 is a plan view illustrating the first member according to one embodiment of the present invention.
FIG. 12 is a cross-sectional view taken along line B-B of FIG. 11.
FIG. 13 is a flowchart illustrating a process of a method for correcting for loss of a chest compression depth of a patient according to one embodiment of the present invention.
FIG. 14 is a diagram for explaining a chest compression start position of the patient according to one embodiment of the present invention.
FIG. 15 is a diagram illustrating the shape of a chest when a lower end of a cap is in contact according to one embodiment of the present invention.
FIG. 16 is a diagram illustrating the shape of the chest in a state where loss of the chest compression depth occurs during the process of the chest compression using the cap according to one embodiment of the present invention.
FIG. 17 is a diagram for explaining a band connected to a base plate according to one embodiment of the present invention.
FIG. 18 is a usage state diagram of the band according to one embodiment of the present invention.

### [Best Mode]

Hereinafter, with reference to the attached drawings, embodiments of the present invention will be described in detail so that those skilled in the art can easily implement the present invention. However, since the description of the present invention is merely an embodiment for structural and functional explanation, the scope of the rights of the present invention should not be construed as being limited by the embodiments described in the text. That is, since the embodiments can be variously modified and can have various forms, the scope of the rights of the present invention should be understood to include equivalents that can realize the technical idea. In addition, the purpose or effect presented in the present invention does not mean that a specific embodiment must include all of them or only such effects, and therefore the scope of the rights of the present invention should not be understood as being limited thereby.

The meanings of terms described in the present invention should be understood as follows.

The terms "first", "second", or the like are intended to distinguish one component from another, and the scope of the right should not be limited by these terms. For example, the first component may be referred to as the second component, and similarly, the second component may also be referred to as the first component. When a component is referred to as being "connected" to another component, it should be understood that it may be directly connected to the other component, but there may also be another component therebetween. Meanwhile, when a component is referred to as being "directly connected" to another component, it should be understood that there is no other component in between. Moreover, other expressions that describe the relationship between components, such as "between" and "directly between" or "adjacent to" and "directly adjacent to", should be interpreted in the same way.

A singular expression should be understood to include the plural expression unless the context clearly indicates otherwise, and the terms "include" or "have" should be understood to specify the presence of a stated feature, number, step, operation, component, part, or combination thereof, but not to exclude the possibility of the presence or addition of one or more other features, numbers, steps, operations, components, parts, or combinations thereof.

All terms used herein, unless otherwise defined, have the same meaning as commonly understood by a person of ordinary skill in the art to which the present invention belongs. Terms defined in commonly used dictionaries should be interpreted as having a meaning consistent with the contextual meaning of the relevant art, and shall not be interpreted as having an ideal or overly formal meaning unless explicitly defined in the present invention.

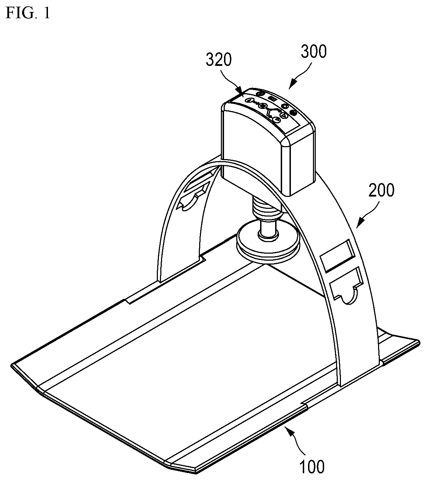

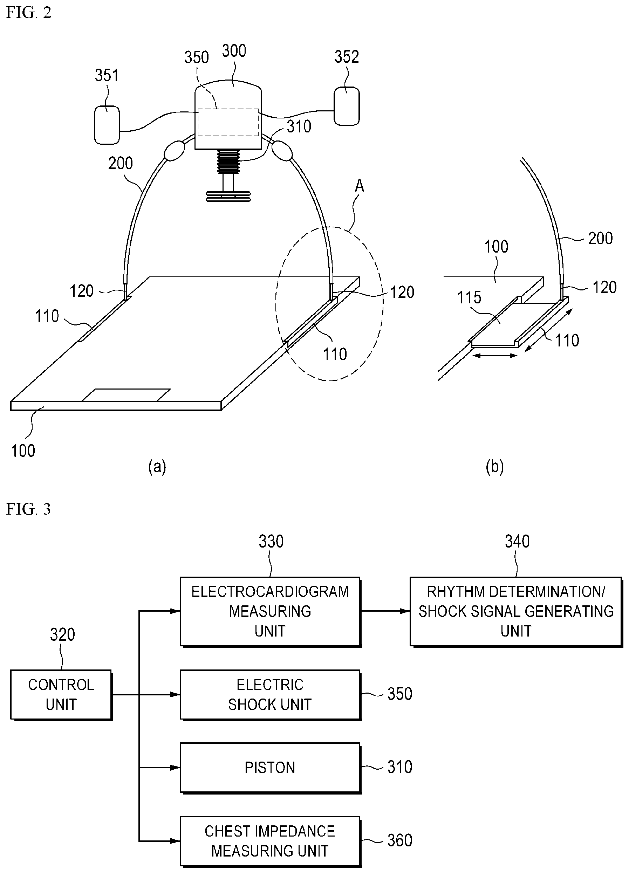

FIG. 1 is a perspective view illustrating a partial configuration of an emergency treatment device for chest compression and defibrillation of a patient according to one embodiment of the present invention, FIG. 2 is a schematic diagram illustrating the partial configuration of the emergency treatment device for the chest compression and defibrillation of the patient illustrated in FIG. 1, and FIG. 3 is a block diagram illustrating a unit equipped in a hood according to one embodiment of the present invention.

Referring to FIGS. 1 to 3, an emergency treatment device for chest compression and defibrillation of a patient (hereinafter, referred to as an "emergency treatment device") according to one embodiment of the present invention includes a base plate 100, a support 200, and a hood 300.

The base plate 100 is formed in a form to support a back of a patient suffering from a disease such as acute cardiac arrest or ventricular fibrillation, and is provided with a sliding guide 110 for slidingly moving the support 200 and the hood 300 to which a cardiopulmonary resuscitation device and an automated external defibrillator are combined, a stopper 120 for fixing the positions of the support 200 and the hood 300, and a band 130 for fixing an arm of the patient by wrapping around the upper portion of the arm of the patient including the elbow.

In the base plate 100, an internal space into which a frame 115 provided in the sliding guide 110 can be inserted to adjust the height of a piston 310 is formed on a side portion of the base plate 100.

The sliding guides 110 are provided on both sides of the base plate 100, and one end and the other end of the support 200 are coupled to be slidable, allowing the support 200 to slide forward or backward.

As illustrated in (b) of FIG. 2, which is an enlarged view of an area A illustrated in (a) of FIG. 2, the sliding guide 110 is provided with the frame 115 that is inserted into or withdrawn from the inside of the base plate 100, thereby allowing the distance between both ends of the support 200 to be adjusted, thereby adjusting the height of the piston 310.

In this case, the reason why the height of the piston 310 is adjusted is to prevent a situation in which a chest compression point of a specific patient cannot be compressed by the piston 310 because each patient has a different body shape.

The stopper 120 is provided in the sliding guide 110 and is formed in a form that can be connected to one end and the other end of the support 200 to connect one end and the other end of the support 200, and fixes the positions of the support 200 and the hood 300 through connection to one end and the other end of the support 200.

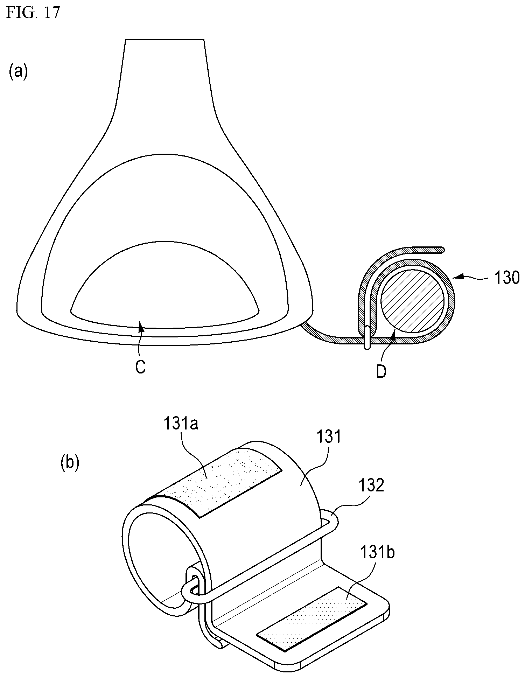

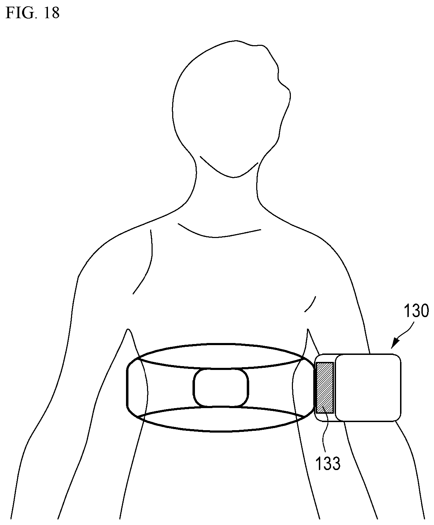

As illustrated in FIGS. 17 and 18, the band 130 includes a cuff 131 for fixing an arm D of the patient by wrapping the upper portion of the arm including the elbow of the patient while the chest compression is performed in a state where the back of the patient is supported by the base plate 100.

The cuff 131 has a D-ring 132 and an opening 133, and wraps around the arm D of the patient on the inside by a structure passing through the D-ring 132, and velcros 131a and 131b that can be attached and detached from each other may be provided on one side so that the cuff is in close contact with the arm D of the patient and the state of wrapping the arm D of the patient is maintained.

Although not illustrated in the drawings, the cuff 131 is connected to the base plate 100 by being wound around a shaft formed in the lower portion of the base plate 100, and, preferably, a pair of cuffs is provided on the side portions of the base plate 100 to wrap both arms D of the patient.

In addition, the cuff 131 is formed with the opening 133 having a diameter into which the user's hand can be inserted so that the user can simultaneously hold the base plate 100 and the cuff 131. Moreover, the user may move the emergency treatment device to a desired location by holding the base plate 100 to which the cuff 131 is connected by inserting his/her hand into a groove formed at the lower portion of the base plate 100 so that the axis of the base plate 100 is provided, although not illustrated in the drawing, together with the opening 133.

Meanwhile, although the emergency treatment device of FIG. 18 does not include the base plate 100 for supporting the back of the patient, it would be preferable to understand that it is an emergency treatment device according to one embodiment of the present invention that includes the base plate 100.

The support 200 is coupled to the sliding guide 110 to move a lower end of the piston 310 to a position where the lower end compresses the chest compression point of the patient, and in one embodiment of the present invention, the shape for supporting the hood 300 may be arched, but is not limited thereto.

One end and the other end of the support 200 are movably coupled to the pair of sliding guides 110, and thus, the support can slide forward or backward with the sliding guide 110 as an axis, or a distance between both ends of the support may be adjusted by inserting and withdrawing the frame 115.

It is preferable that the forward and backward sliding movement of the support 200 and the distance adjustment between both ends of the support are performed before the piston 310 compresses the chest compression point of the patient, and when the support 200 moves to a position for the piston 310 to compress and relax the chest compression point of the patient, one end and the other end are fastened by the pair of stoppers 120.

The support 200 is configured in a form in which one end and the other end are attached to and detached from the pair of sliding guides 110, and can be attached to and detached from the pair of sliding guides 110. Through attachment and detachment, the support is attached to and detached from the base plate 100 together with the hood 300 and may be used as a separate device.

The hood 300 is coupled to one side of the support 200, more specifically, to the center portion (arch crown) of the arch-shaped support 200, and is connected to the piston 310 for compressing the chest compression point of the patient, and the components of the hood 300 are as follows.

Referring to FIG. 3, the hood 300 may be equipped with a control unit 320, an electrocardiogram measuring unit 330, a rhythm determination/shock signal generating unit 340, an electric shock unit 350, and a chest impedance measuring unit 360, in addition to the piston 310 described above, and the components may be exposed to the outside or installed inside the hood 300.

The piston 310 is separated from the chest of the patient before compressing the chest compression point of the patient, and may repeat a process of being operated by the control unit 320 to compress the chest compression point of the patient and then being separated to relax the chest compression point of the patient.

The piston 310 may be operated based on a compression continuous mode that continuously compresses the chest compression point of the patient according to a chest compression mode set through the control unit 320, or a compression 30 : 2 mode that performs chest compression and artificial respiration of the patient simultaneously by performing 2 artificial respirations after performing 30 chest compressions of the patient, and thus, the chest compression-based emergency treatment may be provided to the patient.

The piston 310 may be operated alternately or simultaneously with the electric shock unit 350 by the control unit 320 to provide cardiopulmonary resuscitation and electric shock-based emergency treatment to the patient.

In addition, the piston 310 may directly compress the chest compression point of the patient, but is not limited thereto, and a cap 700 for directly compressing the chest compression point of the patient may be detachably attached to the lower end, and the cap 700 may be made of a material such as silicone to alleviate shock during the process of compressing the chest compression point of the patient.

That is, the hood 300 is configured to couple the cap 700 with the piston 310 or the lower end of the piston 310 to compress the chest compression point of the patient through the cap 700 so that cardiopulmonary resuscitation may be performed. Hereinafter, the emergency treatment device of the present invention will be described in detail as the lower end of the piston 310 compresses the chest compression point of the patient.

Meanwhile, the emergency treatment device of the present invention may provide chest compression and electric shock to a patient alternately or simultaneously through one system depending on the operation timing of the piston 310 and the electric shock unit 350, and therefore has an excellent versatility effect compared to conventional cardiopulmonary resuscitation devices and automated external defibrillators.

The control unit 320 may control the operations of the piston 310, electrocardiogram measuring unit 330, rhythm determination/shock signal generating unit 340, electric shock unit 350, and chest impedance measuring unit 360, which are components provided in the hood 300, and a plurality of buttons may be provided for this purpose.

The plurality of buttons is not illustrated in the drawing, but as a specific example, the plurality of buttons may include a power button for turning the emergency treatment device on/off, a control button for setting the mode of the emergency treatment device to an automatic cardiac shock mode and/or a chest compression mode, a stop button for stopping the operation of the piston 310, a compression mode setting button for causing the piston 310 to perform chest compression (CPR) of the patient or setting the chest compression mode of the piston 310, a compression depth setting button for setting the chest compression depth of the piston 310, a compression count setting button for setting the chest compression count of the piston 310, a shock button for operating the electrocardiogram measuring unit 330, the rhythm determination/shock signal generating unit 340, and the electric shock unit 350, and an adaptive length change button for selecting whether to update the chest compression start position of the patient and correct for loss of the chest compression depth.

The control unit 320 performs a self-test to determine whether the settings are initialized and normal operation is possible when an input signal is input to the power button and the power of the emergency treatment device is turned on. When the emergency treatment device is on, in a case where the input signal is input to the power button again, the settings are initialized and the power of the emergency treatment device is turned off.

The control unit 320 controls the operation of the piston 310 so that the chest of the patient is repeatedly compressed and relaxed when the chest compression mode set through the compression mode setting button is the compression continuous mode. Meanwhile, when the chest compression mode set through the compression mode setting button is the compression 30 : 2 mode, the operation of the piston 310 may be controlled so that two artificial respirations are performed after the chest of the patient is compressed 30 times.

The control unit 320 may control the operation of the piston 310 so that the chest of the patient is compressed to at least one depth of 4 cm, 4.5 cm, 5 cm, and 5.5 cm when the input signal is input to the compression depth setting button, and further, may control the operation of the piston 310 so that the chest of the patient is compressed to 5 cm when the input signal is input to the compression depth setting button in an initialization state, thereafter, the chest of the patient is compressed to 5.5 cm when the signal is input, the chest of the patient is compressed to 4 cm when the signal is input again, and the chest of the patient is compressed to 4.5 cm when the signal is input again.

The control unit 320 may control the operation of the piston 310 so that the chest of the patient is compressed at least one of 100, 110, and 120 times per minute when the input signal is input to the compression count setting button, and further, may control the operation of the piston 310 so that the chest of the patient is compressed 110 times when the input signal is input to the compression count setting button in the initialization state, the chest of the patient is compressed 120 times when the signal is input again, and the chest of the patient is compressed 100 times when the signal is input again.

The electrocardiogram measuring unit 330 is connected to the rhythm determination/shock signal generating unit 340 and the control unit 320, and when operated by the control unit 320, detects an electrocardiogram signal from the patient, amplifies the signal, removes noise from the amplified electrocardiogram signal, converts the electrocardiogram signal into a digital signal, and transmits the digital signal to the rhythm determination/shock signal generating unit 340.

The electrocardiogram measuring unit 330 may be equipped with an amplifier that amplifies the electrocardiogram signal in analog form when the electrocardiogram signal is detected, a filter that removes noise from the electrocardiogram signal amplified by the amplifier, and an A/D converter that converts the electrocardiogram signal of which noise has been removed by the filter into a digital signal.

The electrocardiogram measuring unit 330 is provided on one side of the hood 300 to detect the electrocardiogram signal in analog form from the patient, and is formed of an electrode or pad that can be attached to the patient, thereby detecting the electrocardiogram signal from the patient supporting his/her back by the base plate 100.

Here, the process of detecting the electrocardiogram signal of the patient through electrodes or pads is conventional, so descriptions thereof will be omitted for convenience.

The rhythm determination/shock signal generating unit 340 is built into the hood 300 and is connected to the electrocardiogram measuring unit 330 so as to operate together when the electrocardiogram measuring unit 330 is operated by the control unit 320, thereby receiving a digital signal from the electrocardiogram measuring unit 330.

When the rhythm determination/shock signal generating unit 340 receives the digital signal from the electrocardiogram measuring unit 330, the rhythm determination/shock signal generating unit analyzes the electrocardiogram of the patient using the digital signal to determine whether the electrocardiogram of the patent is a shockable rhythm or a non-shockable rhythm, generates an electric shock signal when the electrocardiogram of the patient is determined to be the shockable rhythm, and transmits the electric shock signal to the electric shock unit 350 when the electric shock unit 350 is operated.

The electric shock unit 350 is connected to the rhythm determination/shock signal generating unit 340, and when the electrocardiogram of the patient is determined to be the shockable rhythm by the rhythm determination/shock signal generating unit 340, the electric shock unit receives an electric shock signal from the rhythm determination/shock signal generating unit 340 when operated by the control unit 320.

The electric shock unit 350 emits high-voltage energy from a pair of first electrodes 351 and a pair of second electrodes 352 when receiving the electric shock signal, thereby applying the electric shock to the patient.

The pair of first electrodes 351 and the pair of second electrodes 352 are preferably attached to the chest of the patient so as to apply the electric shock to the patient. As a specific example, the pair of first electrodes 351 may be attached below the right collarbone of the patient to apply the electric shock to the patient, and the pair of second electrodes 352 may be attached to the armpit next to the left nipple of the patient to apply the electric shock to the patient.

In addition, the pair of first electrodes 351 and the pair of the second electrodes 352 may be attached to or detached from the electric shock unit 350 through a cable and electrically connected to the electric shock unit 350 to emit high-voltage energy.

Moreover, the pair of first electrodes 351 and the pair of second electrodes 352 are described as components of the hood 300 in one embodiment of the present invention, but are not limited thereto, and may be components provided on the base plate 100 and may be electrically connected to the electric shock unit 350 through a cable to emit high-voltage energy.

The chest impedance measuring unit 360 measures the chest impedance of the patient, and does not limit the method of measuring the chest impedance, but in a specific example, a pair of first electrodes 351 that are current output electrodes capable of outputting current as well as emitting high-voltage energy and a pair of second electrodes 352 that are voltage detection electrodes capable of detecting voltage may be used to continuously measure the chest impedance value during the compression and relaxation of the chest of the patient.

Meanwhile, the control unit 320 may calculate whether the pair of first electrodes 351 and the pair of second electrodes 352 are properly attached to the patient within a chest impedance range of the patient measured by the chest impedance measuring unit 360, and calculate a profile of a biphasic electric shock waveform using a chest impedance value 421a when the chest of the patient is compressed and a chest impedance value 421b when the chest of the patient is relaxed.

In one embodiment of the present invention, the emergency treatment device may further be equipped with a storage unit 400 that stores information required during the emergency treatment process for the patient, the storage unit 400 may be composed of a first storage unit 410 and a second storage unit 420, and the information stored in each of the storage units 410 and 420 is as follows.

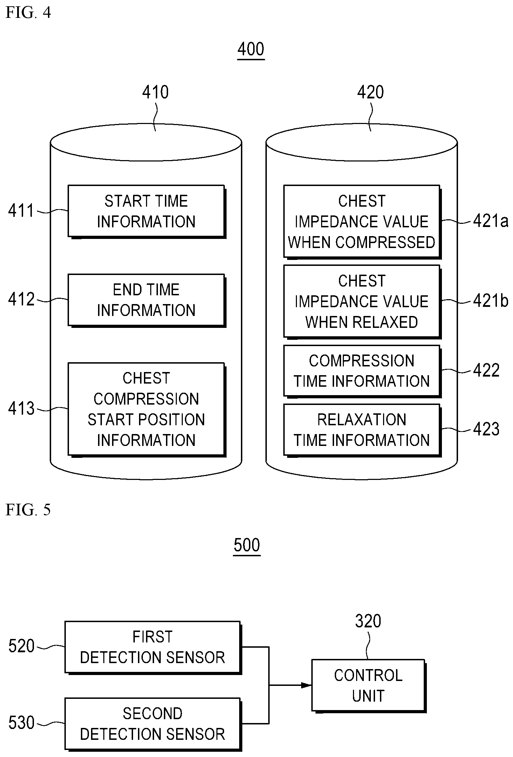

FIG. 4 is a diagram illustrating the storage unit and the stored information provided in the emergency treatment device for chest compression and defibrillation of the patient according to one embodiment of the present invention.

Referring to FIG. 4, the first storage unit 410 pre-stores start time information 411 for starting the electrocardiogram analysis of the patient, end time information 412 for ending the electrocardiogram analysis of the patient, and information 413 on the chest compression start position to which the piston 310 will return after completing chest compression and relaxation of the patient.

The second storage unit 420 stores a chest impedance value 421a measured by the chest impedance measuring unit 360 when the chest of the patient is compressed, a chest impedance value 421b when the chest of the patient is relaxed, compression time information 422 when the piston 310 compresses the chest compression point of the patient, and relaxation time information 423 when the chest compression point of the patient is relaxed.

Although the storage unit 400 is described as including the first storage unit 410 and the second storage unit 420 in one embodiment, it is not limited thereto, and the first storage unit 410 and the second storage unit 420 may be provided as one storage unit in which each piece of information may be stored or pre-stored.

Meanwhile, the control unit 320 may calculate an average value of the chest impedances during the compression and relaxation of the chest of the patient using the compression time information and relaxation time information stored in the second storage unit 420, and generate an optimal biphasic electric shock waveform profile during the compression and relaxation of the chest of the patient based on each average value of chest impedances.

In addition, when a change occurs in the chest impedance values 421a and 421b stored in the second storage unit 420 during the compression and relaxation of the chest of the patient, the control unit 320 may calculate an absolute value of a difference between the chest impedance values 421a and 421b continuously measured through the chest impedance measuring unit 360 during the compression and relaxation of the chest of the patient and an average value of a certain number of chest impedance values among the chest impedance values 421a and 421b stored in the second storage unit 420.

Moreover, when the absolute value of the difference with respect to the average value of the calculated chest impedances is equal to or more than a preset absolute value, the control unit 320 may exclude the chest impedance average value stored in the second storage unit 420 before the change occurs, replace with the chest impedance values 421a and 421b during the compression and relaxation of the chest of the patient measured by the chest impedance measuring unit 360 after excluding the average value of the chest impedance, and generate the profile of the optimal biphasic electric shock waveform during the compression and relaxation of the chest of the patient by using the chest impedance values 421a and 421b.

That is, according to one embodiment of the invention, the chest impedance value 421a during the chest compression and a chest impedance value 421b during the chest relaxation are each obtained, and an average value of each is calculated. Moreover, using each of the calculated chest impedance average values, when the chest compression is performed at the time of applying the electric shock, an electric shock waveform profile corresponding to the average impedance value during the chest compression is generated, and when the chest relaxation is performed at the time of applying the electric shock, an electric shock waveform profile corresponding to the average impedance value during the chest relaxation is generated, and thus, an optimal electric shock waveform profile is generated for the patient.

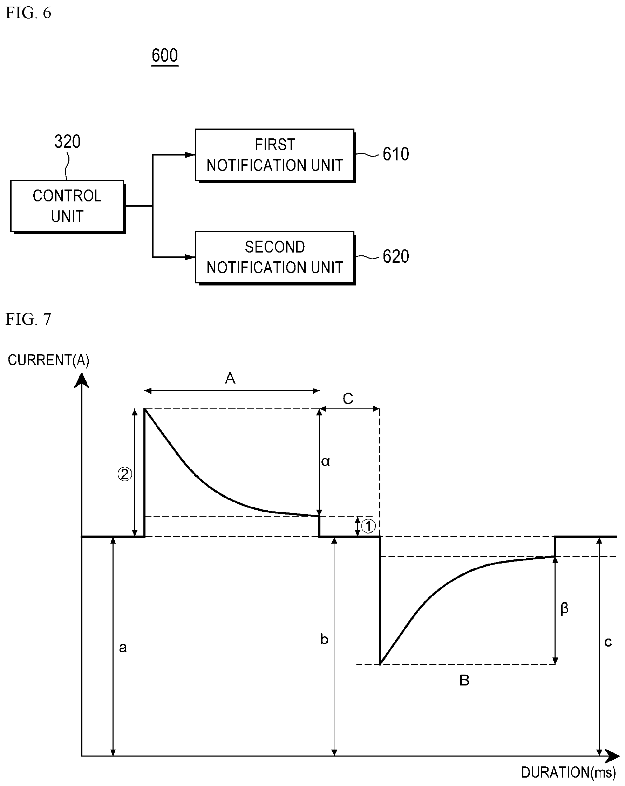

Here, the profile of the optimal biphasic electric shock waveform generated in the control unit 320 will be described in detail through FIG. 7 and [Table 1] below.

**[Table 1]**

| Impedance | First phase section (ms) | Second phase section (ms) | Peak current (A) | Discharge energy (J) | Energy accuracy(J) |
|---|---|---|---|---|---|
| 25 | 2.4 | 2.4 | 70.5 | 201 | 200(±15%) |
| 50 | 4.4 | 4.4 | 35.6 | 200.7 | 200(±15%) |
| 75 | 6.5 | 6.5 | 25 | 203 | 200(±15%) |
| 100 | 8.8 | 8.8 | 18.9 | 204 | 200(±15%) |
| 125 | 10.7 | 10.7 | 15.09 | 202.6 | 200(±15%) |
| 150 | 12.7 | 12.7 | 12.81 | 203.8 | 200(±15%) |
| 175 | 14.8 | 14.8 | 11.15 | 204.7 | 200(±15%) |
| 25 | 2.4 | 2.4 | 64.5 | 147.8 | 150(±15%) |
| 50 | 4.4 | 4.4 | 32.7 | 149.7 | 150(±15%) |
| 75 | 6.3 | 6.3 | 22.5 | 151.5 | 150(±15%) |
| 100 | 8.8 | 8.8 | 15.9 | 148.1 | 150(±15%) |
| 125 | 10.7 | 10.7 | 13.0 | 149 | 150(±15%) |
| 150 | 12.7 | 12.7 | 11.0 | 148.2 | 150(±15%) |
| 175 | 15.0 | 15.0 | 9.5 | 148.8 | 150(±15%) |

Referring to FIG. 7, the profile of the biphasic electric shock waveform includes positive and negative waveforms, and represents a current value and duration applied when applying an electric shock to a patient. In addition, Table 1 illustrates the duration (ms) of the first phase section, the duration (ms) of the second phase section, the peak current (A), and the discharging energy (J) calculated through these, which are provided according to the average impedance value measured when the chest of the patient is relaxed or contracted. In Table 1, the discharging energy (J) is discharging energy for an adult patient, and preferably 200J may be applied to an adult patient, but is not limited thereto. Referring again to FIG. 7, the profile of the biphasic electric shock waveform has the duration (ms) on the x-axis and the current (A) on the y-axis, and FIG. 7 illustrates a symmetric profile according to one embodiment of the present invention, but is not necessarily limited thereto, and may be configured as an asymmetric profile.

When the present invention is actually implemented, the duration (ms) of the first phase section, the duration (ms) of the second phase time, and the peak current (A) value according to each impedance value illustrated in Table 1 are values preset to the control unit 320, and with the values of the duration (ms) of the first phase section, the duration (ms) of the second phase time, and the peak current (A) corresponding to the average impedance value measured at the time of the compression and relaxation of the chest, an electric shock is applied to the patient according to the biphasic electric shock waveform profile.

In FIG. 7, a is the current value before the first phase section, b is the current value between the first and second phase sections, and c is the current value after the second phase section, and the values of a, b, and c may all be the same. In addition, α is the reduction amount that is reduced while the peak current is lowered in the first phase section, and similarly, β is the reduction amount that is reduced while the peak current is lowered in the second phase section, and the values of α and β may be the same. In addition, A is the duration in the first phase section, and B is the duration in the second phase section, and the values of A and B may be the same. In addition, ① is the difference value between the current value immediately before the end of the first phase section and the current value immediately before the start of the second phase section, ② is the absolute value of the peak current in the first phase section, and the sum of ① and α may be ②.

In one embodiment of the present invention, the emergency treatment device may further be equipped with a sensor 500 for detecting information during the emergency treatment process of the patient, and the sensor 500 may include a first detection sensor 520 and a second detection sensor 530.

FIG. 5 is a diagram illustrating a sensor equipped in the emergency treatment device for chest compression and defibrillation of the patient according to one embodiment of the present invention.

Referring to FIG. 5, the sensor 500 detects the current position of the piston 310 in real time through the first detection sensor 520 to generate current position information, and transmits the current position information of the piston 310 to the control unit 320 in real time.

Meanwhile, the control unit 320 controls the operation of the piston 310 using information from the first detection sensor 520 and the second detection sensor 530 before analyzing the electrocardiogram of the patient. An example of the process of controlling the operation of the piston 310 using information on the current position of the piston 310 is as follows.

The control unit 320 moves the lower end of the piston 310 to the chest compression start position so that the lower end of the piston 310 does not come into contact with the chest of the patient before the electrocardiogram of the patient is analyzed, and when the lower end of the piston 310 is moved to the chest compression start position, the control unit determines whether the current position information of the piston 310 received in real time matches the chest compression start position and places the lower end of the piston 310 relatively higher than the chest compression start position, thereby putting the piston 310 into a standby state before the piston compresses the chest compression point of the patient.

In addition, the control unit 320 may control the operation of the piston 310 so that the chest of the patient is compressed and relaxed according to the settings of the chest compression mode, chest compression depth, and chest compression count by the plurality of buttons from the chest compression start position after analyzing the electrocardiogram of the patient.

Moreover, before the piston 310 compresses and relaxes the chest compression point of the patient, the control unit 320 considers the distance between the lower end of the piston 310 and the chest of the patient as a preset distance (for example, 0 to 2 cm) so that the chest of the patient is compressed when the piston 310 is lowered by the chest compression depth set by the compression depth setting button. That is, the chest compression start position of the patient means the position of the piston 310 that should be positioned to compress the chest of the patient in the process of compressing and relaxing the chest of the patient.

In addition, in one embodiment, the control unit 320 may optimize the chest compression start position of the patient by updating the chest compression start position of the patient at regular intervals (for example, the chest compression mode, 2 minutes and 30 seconds after settings of chest compression depth and chest compression counter are completed). The updating of the chest compression start position of the patient at regular intervals in this manner is because the chest of the patient may sink through the compression and relaxation of the chest, or the patient may move while supporting his or her back by the base plate 100, and thus the optimization of the chest compression start position of the patient may not be achieved.

Moreover, the control unit 320 may receive electrical information (for example, power) used from the motor for driving the piston 310 and current piston position information, and when a change in the chest compression start position of the patient is detected, the control unit may update the chest compression start position of the patient. In this case, the sensors that detect the electrical information, the rotation speed of the motor, and the piston movement distance information may be the first detection sensor 520 and the second detection sensor 530.

Referring again to FIG. 5, the first detection sensor 520 detects the electrical information used from the motor and transmits the electrical information to the control unit 320.

The first detection sensor 520 may be equipped in the driving circuit of the motor, detect the position of the lower end of the piston 310 and then transmit the detected position to the control unit 320 in real time.

In this case, the control unit 320 may receive the lower end position value of the piston 310 from the first detection sensor 520 to determine the current position of the piston 310. At this time, when the cap 700 is coupled to the lower end of the piston 310 as described above to compress the chest of the patient, the control unit 320 may calculate the lower end position value of the piston 310 by considering the value of the pre-input width of the first housing 710 positioned between the chest of the patient.

As an example of a method in which the first detection sensor 520 detects the position of the lower end of the piston, when the piston 310 descends through the rotation of the motor, the power consumption of the motor is maintained constant while descending through the air. The power consumption of the motor remains constant while descending through the air, and from the moment the lower end of the piston touches the surface of the chest of the patient, the power consumption of the motor increases. The first detection sensor 520 may detect the power consumption of the motor, recognize the moment when the power consumption increases as the surface of the chest of the patient, and detect the position value of the lower end of the piston at this time and transmit the position value to the control unit 320.

Moreover, the first detection sensor 520 may detect whether the shape of the chest of the patient has changed by using the difference in the electrical information when the lower end of the piston 310 comes into contact with the surface of the chest of the patient at regular intervals, and transmit whether the shape of the chest of the patient has changed to the control unit 320.

In addition, when the cap 700 is fit-coupled into the lower end of the piston 310, the first detection sensor 520 may detect whether the shape of the chest of the patient has changed by using the difference in the electrical information when the lower end of the cap 700 comes into contact with the surface of the chest of the patient, and also transmit whether the shape of the chest of the patient has changed to the control unit 320.

The second detection sensor 530 is equipped in the motor and detects the rotational speed of the motor and the movement distance of the piston, then generates movement distance information of the piston 310 and transmits the piston movement distance information to the control unit 320.

In one embodiment, the sensor 500 is provided with the first detection sensor 520 and the second detection sensor 530, but is not limited thereto. The first detection sensor 520 and the second detection sensor 530 may be provided as a single detection sensor and detect changes in the electrical information, motor rotation speed, and piston movement distance information, respectively.

In one embodiment of the present invention, the emergency treatment device may further be equipped with a notification unit 600 to notify the user of a message for the progress of emergency treatment for the patient, and the notification unit 600 may include a first notification unit 610 and a second notification unit 620.

FIG. 6 is a diagram illustrating a notification unit equipped in the emergency treatment device for chest compression and defibrillation of the patient according to one embodiment of the present invention.

Referring to FIG. 6, the first notification unit 610 outputs a first message to notify that the chest compression start position of the patient has been updated when the chest compression start position of the patient is updated, and a second message to notify the difference value between the existing chest compression start position of the patient before updating and the updated chest compression start position of the patient.

In addition, the first notification unit 610 outputs a third message when the difference value between the existing chest compression start position of the patient and the updated chest compression start position of the patient is equal to or more than a specified threshold value. The specified threshold value, which is a criterion for outputting the third message, is not limited, but may be set to 1 cm in one embodiment of the present invention.

That is, in one embodiment, the first notification unit 610 may output the third message when the difference value between the existing chest compression start position of the patient and the updated chest compression start position of the patient is 1 cm or more, and may output a fourth message to request the user to select the chest compression depth (for example, at least one of 4 cm, 4.5 cm, 5 cm, and 5.5 cm) and the chest compression count (for example, at least one of 100, 110, and 120 times per minute) of the piston 310 when the chest compression start position of the patient is updated.

In this case, the user of the emergency treatment device that performs emergency treatment on the patient may select the chest compression depth and the chest compression count of the piston 310 by inputting a signal to the compression depth setting button and the compression count setting button of the control unit 320 after receiving the fourth message so that the chest compression depth and the chest compression count of the piston 310 are selected, and the control unit 320 may update the chest compression depth and the chest compression count when the chest compression depth and the chest compression count are selected through the compression depth setting button and the compression count setting button.

Moreover, when the change in the shape of the chest of the patient is detected by the first detection sensor 520, the first notification unit 610 may output a fifth message to notify the user that the shape of the chest of the patient has changed.

An emergency treatment device according to one embodiment of the present invention may provide the user with a message identical to the message output from the first notification unit 610 by visually outputting the message through a visual display device including an LED implemented as an LED light board or an LED flashing light and a display when at least one of the first, second, third, fourth, and fifth messages output from the first notification unit 610, which is an auditory display device, is output as a voice.

The second notification unit 620 may output a message to notify the user when the absolute value of the difference with respect to the current calculated chest impedance value using each chest impedance average value during the compression and relaxation of the chest of the patient stored in the second storage unit 420 by the control unit 320 is equal to or greater than a preset absolute value.

Meanwhile, the cap 700 to be attached to the lower end of the piston 310 is mounted on the lower end of the piston 310 and is made of a material with a hardness different from that of the piston 310 made of a hard material, so that the cap may continuously provide a cushioning effect that relieves and distributes the pressure applied to the chest of the patient.

Below, the structure of the cap 700 will be described in detail.

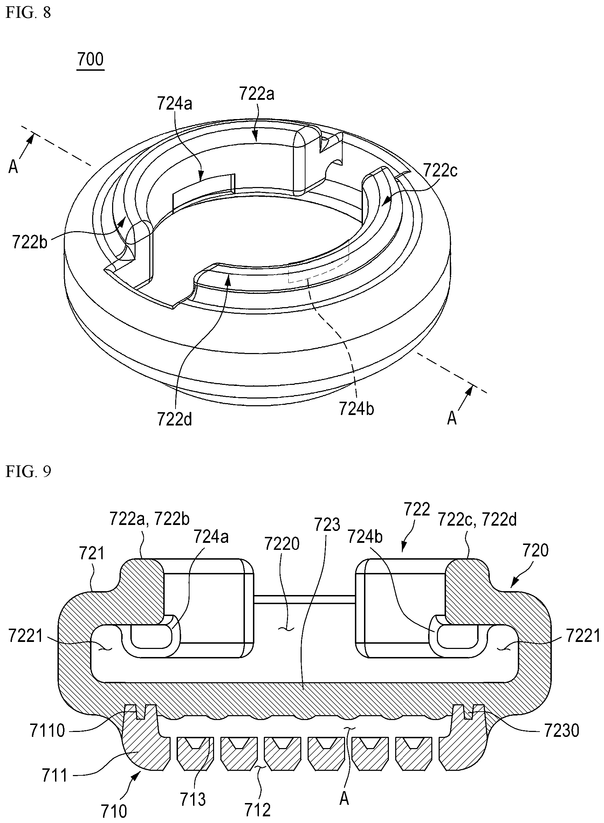

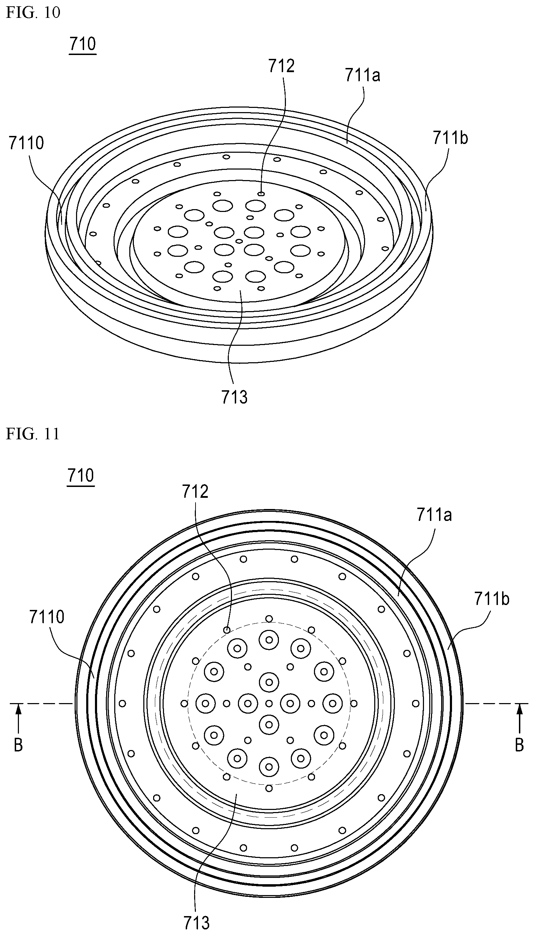

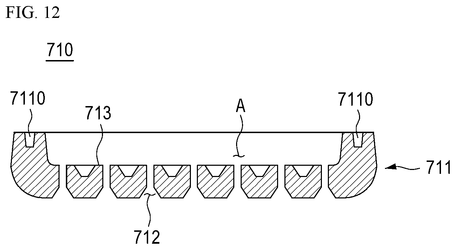

FIG. 8 is a perspective view of a cap according to one embodiment of the present invention, FIG. 9 is a cross-sectional view taken along line A-A of FIG. 8, FIG. 10 is a perspective view illustrating a first member according to one embodiment of the present invention, FIG. 11 is a plan view illustrating a first member according to one embodiment of the present invention, and FIG. 12 is a cross-sectional view taken along line B-B of FIG. 11.

Referring to FIGS. 8 to 12, the cap 700 includes a first member 710 and a second member 720.

The first member 710 may come into direct contact with the chest compression point of the patient and compress the chest of the patient when the piston 310 and the cap 700 are fit-coupled and then the piston 310 is extended toward the chest of the patient.

In addition, the first member 710 is made of at least one of ethylene-vinyl acetate, polyethylene, polyethylene-polypropylene blend, polystyrene, neoprene, chloroprene, and polyurethane, and biocompatible silicone, and due to the characteristics of these materials, the first member may be implemented as a shape that conforms to the shape of the chest of the patient.

Moreover, in the first members 710, the biocompatible silicone may have a hardness of 10 to 30 Shore A, and the other materials may have a hardness of 10 to 20 Asker C. In one embodiment of the present invention, the Asker C hardness may be measured by an Asker hardness tester that applies a predetermined shape of indenter to the surface of a sample with the force of a spring and measures the hardness based on the depth into which the indenter is indented into the sample in a state where the resistance of the sample and the force of the spring are balanced, and the shore hardness measures the height of the rebound when an object with a small diamond fixed to the end of the object is dropped from a certain height.

In addition, the first member 710 has an outer shape formed through a first housing 711 as illustrated in FIGS. 9 to 12, and the first housing 711 may include a plurality of air flow holes 712 and a seating portion 713.

The first housing 711 includes an inner housing 711a and an outer housing 711b integrally formed, and the lower surface comes into contact with the chest of the patient.

The inner housing 711a has the plurality of air flow holes 712 formed on the lower surface so that when pressure is transmitted from the chest of the patient to the lower surface in the process of compressing the chest of the patient, a change in volume occurs through the air flow in an interspace A.

The upper part of the inner housing 711a and the interspace A of the bottom portion 723 may decrease in volume when air flows outward along the air flow hole 712 by the piston 310 that expands in the process of compressing the chest of the patient, and in contrast, when the upper part of the inner housing 711a and the interspace A of the bottom portion 723 are separated from the chest of the patient after the completion of compressing the chest of the patient, the volumes thereof may increase according to the air flowing in through the air flow hole 712.

In the inner housing 711a, the lower surface comes into contact with the chest of the patient in the process of compressing the chest of the patient, and as the piston 310 expands, air flows outward from the interspace A. Accordingly, when the volume of the interspace A decreases and the seating portion 713 provided at the center comes into contact with the lower surface of the bottom portion 723, a negative pressure is generated in the interspace A, and when the piston 310 is contracted after the negative pressure is generated in the interspace A, the lower surface that comes into contact with the chest compression point of the patient may move upward while pulling the chest of the patient.

The first housing 711 has a protruding member insertion hole 7110 to which a protruding member 7230 to be described later can be fit-coupled at the boundary between the inner housing 711a and the outer housing 711b.

The protruding member insertion hole 7110 may be formed in a circular shape at the boundary between the inner housing 711a and the outer housing 711b to enable fit-coupling of the protruding member 7230.

In addition, the inner housing 711a and the outer housing 711b have side walls that form the protruding member insertion hole 7110 protruding upward, thereby creating the interspace A between the inner housing 711a and the bottom portion 723 in the fit-coupling structure of the first member 710 and the second member 720.

The first housing 711 needs to conform to the chest of the patient when compressing the chest of the patient, and accordingly, the lower surface of the inner housing 711a that comes into contact with the chest compression point of the patient is preferably made of biocompatible silicone among applicable materials that easily conforms to the chest of the patient, thereby continuously providing a cushioning effect that relieves and distributes the pressure applied to the chest of the patient, thereby preventing rib fractures and hemothorax from occurring during emergency treatment.

In addition, in the first housing 711, the air in the interspace A flows outward from the plurality of air flow holes 712, the volume of the interspace A is reduced, and when the seating portion 713 comes into contact with the lower surface of the bottom portion 723 of the second member 720, the interspace A is brought into a negative pressure state.

Moreover, in the first housing 711, the inner housing 711a and the outer housing 711b may be implemented in a bellows shape so that the volume of the interspace A can be changed according to the air flow.

The second member 720 is fit-coupled with the piston 310 and comes into contact with the lower end of the piston 310, and may be fit-coupled with the first member 710.

The second member 720 may be made of one of polyurethane and polypropylene and biocompatible silicone, and thus may be different in material from the first member 710.

In addition, the second member 720 may have a hardness of 40 to 60 Shore A in the case of biocompatible silicone, and a hardness of 25 to 30 Asker C in the case of other materials, so that the hardness may be different from that of the first member 710.

Moreover, in the second member 720, a second housing 721, a piston fitting portion 722, and bottom portion 723 may be integrally formed as illustrated in FIGS. 9 to 12.

The second housing 721 forms the outer shape of the second member 720 and is integrally formed with the piston fitting portion 722 and the bottom portion 723.

The piston fitting portion 722 is formed integrally with the second housing 721 and forms a piston fitting hole 7220 so that the piston 310 is fit-coupled to the upper portion of the second member 720.

In addition, the piston fitting portion 722 is a part that is formed by being bent from the second housing 721 to form the piston fitting hole 7220 into which a fastening member (not illustrated) of the piston 310 can be inserted, and may include a first piston fitting portion 722a, a second piston fitting portion 722b, a third piston fitting portion 722c, and a fourth piston fitting portion 722d.

In addition, the piston fitting portion 722 has a pair of grooves 724a and 724b formed between the first piston fitting portion 722a and the second piston fitting portion 722b and between the third piston fitting portion 722c and the fourth piston fitting portion 722d, and when the piston 310 is fit-coupled into the piston fitting hole 7220, a fastening member formed on a portion of the outer peripheral surface of the piston 310 may be inserted into the grooves 724a and 724b.

That is, the piston 310 may be fastened to the cap 700 by the lower end portion being in contact with the bottom portion 723 and the fastening member being inserted into the pair of grooves 724a and 724b.

Moreover, the piston fitting portion 722 may have a lower portion that may expand (or extend) toward the outside of the second housing 721 so that the fastening member of the piston 310 may be inserted into or withdrawn from a pair of grooves 724a and 724b in the process of coupling and decoupling between the fastening member of the piston 310 and the grooves 724a and 724b, and for this purpose, an expansion space 7221 may be formed in the space between the piston fitting portion and the second housing 721.

The bottom portion 723 is formed integrally with the second housing 721 as the lower surface of the second member 720, and when the piston 310 is fit-coupled to the piston fitting hole 7220, the upper surface of the bottom portion comes into contact with the lower end of the piston 310.

In addition, the protruding member 7230 is provided on the lower surface of the bottom portion 723 and fit-coupled to the protruding member insertion hole 7110, and thus, the fit-coupling between the first member 710 and the second member 720 is realized.

The protruding member 7230 may be protruded in a circular shape from the lower surface of the bottom portion 723 to be fit-coupled to the protruding member insertion hole 7110.

Hereinafter, the process of a method (S100) for correcting for loss of a chest compression depth of a patient performed by the emergency treatment device according to one embodiment of the present invention will be described in detail.

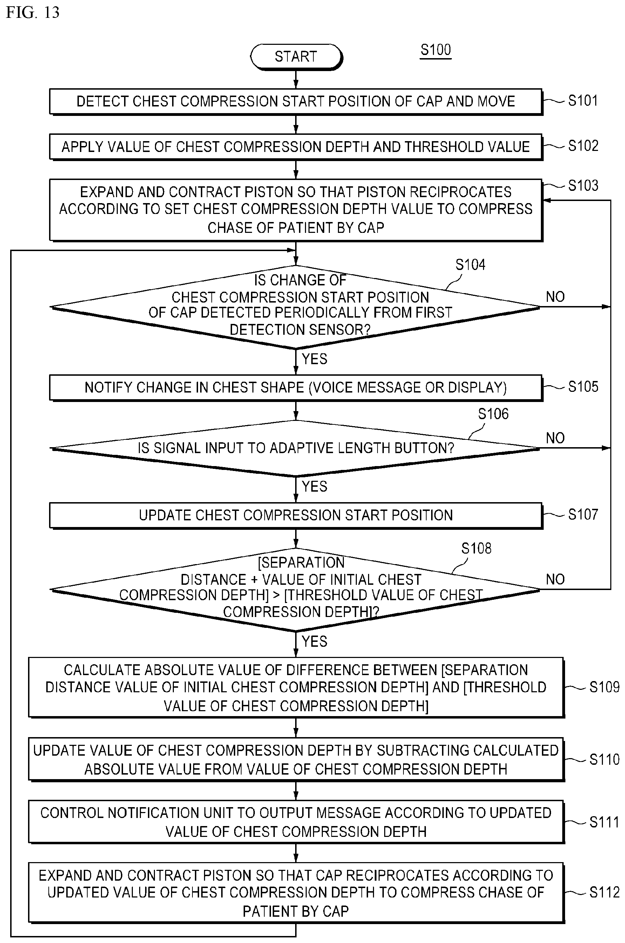

Referring to FIG. 13, the user of the emergency treatment device may set the value of the chest compression start position of the patient to start compressing the chest of the patient with the lower end of the piston 310 positioned above the chest of the patient using the control unit 320, and then expand or contract the piston 310 so that the lower end of the piston 310 moves to the chest compression start position according to the value of the chest compression start position of the patient (S101).

Here, the expansion of the piston 310 means a descending process to compress the chest of the patient, and the contraction means an ascending process to relax the chest of the patient.

Additionally, the chest compression start position movement step (S101) of the patient may be performed through the first detection sensor 520 as described above.

After the chest compression start position movement step (S101) of the patient, the user may apply a value L of the chest compression depth, which is the movement length value of the piston 310 toward the upper side of the base plate 100 from the chest compression start position of the patient, and a threshold value T of the chest compression depth using the control unit 320 (S102).

After the step of applying the value of the chest compression depth and the threshold value of the chest compression depth (S102), the control unit 320 expands and contracts the piston 310 so that the piston reciprocates according to the set chest compression depth to compress the chest of the patient by the cap 700 (S103).

The control unit 320 may periodically detect whether the chest compression start position of the patient has changed from the first detection sensor 520 while the chest of the patient is compressed (S104).

In this case, when the change in the chest compression start position of the patient is not detected by the first detection sensor 520 and therefore the change in the chest compression start position of the patient is not necessary (S104-NO), the control unit 320 maintains the chest compression step of the patient (S103) so that the chest of the patient is compressed.

In contrast, when the change in the chest compression start position of the patient is detected by the first detection sensor 520 and the chest compression start position of the patient needs to be changed (S104-YES), the control unit 320 may notify the user of the change in chest shape due to the need to change the chest compression start position of the patient through the first notification unit 610 which is an auditory display device, or a visual display device including an LED implemented as an LED light board or LED flashing light and a display (S105).

In this way, after audibly/visually notifying the user of the change in the chest shape (S105), the control unit 320 may determine whether a signal is input to the adaptive length button included in the emergency medical device (S106).

In this case, when no signal is input to the adaptive length button (S106-NO), the control unit 320 maintains the chest compression step of the patient (S103) so that the chest of the patient is compressed.

In contrast, when a signal is input to the adaptive length button (S 106-YES), the control unit 320 updates the chest compression start position of the patient (S107), and calculates and compares the sum of the separation distance X and the value L of the initial chest compression depth with the threshold value T of the chest compression depth (S108).

In this case, when the sum of the separation distance X between the chest of the patient and the cap 700 and the value L of the initial chest compression depth is less than the threshold value T of the chest compression depth (S108-NO), the control unit 320 maintains the chest compression step of the patient (S103) so that the chest of the patient is compressed.

In contrast, when the sum of the separation distance X between the chest of the patient and the cap 700 and the value L of the initial chest compression depth exceeds the threshold value T of the chest compression depth (S108-YES), the absolute value of the difference between the sum of the separation distance X and the value L of the initial chest compression depth and the threshold value T of the chest compression depth may be calculated (S109).

After the absolute value calculation step (S109), the control unit 320 may update the value L of the chest compression depth by subtracting the absolute value from the value L of the chest compression depth (S110).

After the step of updating the value of the chest compression depth (S110), the control unit 320 may control the first notification unit 610 to output the message according to the updated value L of the chest compression depth (S111).

In the first notification unit control step (S111), the message output by the first notification unit 610 may be the first message for notifying that the chest compression start position of the patient has been updated when the chest compression start position of the patient is updated in the first notification unit 610, and the second message for notifying the difference value between the existing chest compression start position of the patient before the update and the updated chest compression start position of the patient, and the control unit 320 may control information on the first and second messages to be output from a visual display device including an LED implemented as an LED light board or LED flashing light and a display, as well as the first notification unit 610.

In addition, in the first notification unit control step (S111), the control unit 320 may transmit information on the first and second messages to the terminal equipped with the user of the emergency treatment device by communicating with the terminal and provide the information to the user. Therefore, the user may check the information on the value L of the chest compression depth and the absolute value updated during or after emergency treatment of the patient via the terminal.

The control unit 320 expands and contracts the piston 310 so that the cap 700 reciprocates according to the updated value L of the chest compression depth after the first and second messages are output once each, thereby allowing chest compression by the cap 700 to perform (S112).

The timing at which the control unit 320 reciprocates the cap 700 is not limited to when the first and second messages are output once from the first notification unit 610, and immediately after the value L of the chest compression depth is updated, the piston 310 may be expanded and contracted so that the cap 700 reciprocates according to the updated value L of the chest compression depth.

Below, the chest shape of the patient during the process of the method (S100) for correcting for the loss of the chest compression depth of the patient will be described in detail.

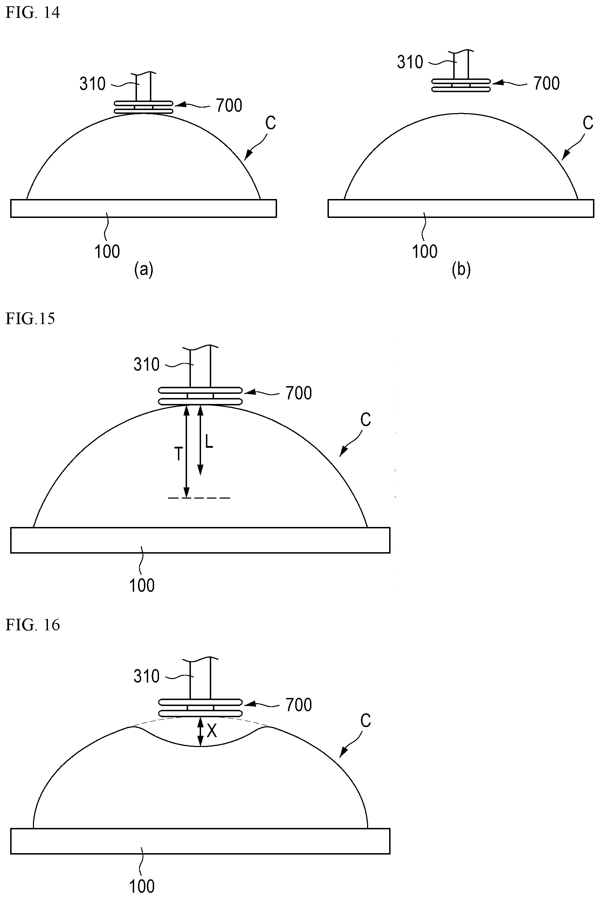

FIG. 14 is a diagram for explaining the chest compression start position of the patient according to one embodiment of the present invention, FIG. 15 is a diagram illustrating a shape of the chest when the lower end of the cap is in contact according to one embodiment of the present invention, and FIG. 16 is a diagram illustrating the shape of the chest in a state where loss of the chest compression depth occurs during the process of the chest compression using the cap according to one embodiment of the present invention.

In the chest compression start position movement step of the patient (S101), the control unit 320 causes the cap 700 to expand from the chest compression start position as illustrated in (a) of FIG. 14 so as to come into contact with a chest C of the patient when the chest compression process is performed alone, and, on the other hand, when the chest compression and electrocardiogram analysis (shockable rhythm or nonshockable rhythm analysis) by the hood 300 are performed in parallel, the piston 310 may be spaced apart from the chest C of the patient by a predetermined distance (for example, 2 cm) as illustrated in (b) of FIG. 14 so that the electrocardiogram analysis is not disturbed by the piston 310 and the cap 700 at the time of the electrocardiogram analysis before and after the chest compression.

Referring to FIG. 15, in the chest compression progress step (S103, S112) by the cap, the piston 310 is expanded by the control unit 320 to move the cap 700 from the chest compression start position to come into contact with the chest C of the patient, and the cap 700 may compress the chest C of the patient by the value L of the chest compression depth set or updated by the control unit 320 through the expansion of the piston 310.

Referring to FIG. 16, in the step (S103, S112) in which the chest compression by the cap is performed, when the cap 700 compresses the chest C of the patient, the shape of the chest C of the patient is deformed as illustrated in FIG. 16. At this time, loss in the value L of the chest compression depth occurs by the separation distance X between the lower end of the cap 700 and the deformed chest C of the patient, so that the chest compression process of the patient may not be performed accurately. Accordingly, the control unit 320 compares and determines the value L of the chest compression depth with the threshold value T of the chest compression depth, and uses the set or updated value L of the chest compression depth to compress the chest of the patient, thereby correcting for the loss of chest compression depth, thereby ensuring that the chest compression process of the patient is performed accurately.

The detailed description of the preferred embodiments of the present invention disclosed above has been provided to enable those skilled in the art to implement and practice the present invention. While the above has been described with reference to preferred embodiments of the present invention, it will be understood by those skilled in the art that various modifications and changes can be made to the present invention without departing from the scope of the present invention. For example, those skilled in the art can utilize each of the configurations described in the above-described embodiments in a manner that combines them. Accordingly, the present invention is not intended to be limited to the embodiments illustrated herein, but is to be accorded the widest scope consistent with the principles and novel features disclosed herein.

The present invention can be embodied in other specific forms without departing from the technical spirit and essential characteristics of the present invention. Therefore, the above detailed description should not be construed as restrictive in all aspects but should be considered illustrative. The scope of the present invention should be determined by reasonable interpretation of the appended claims, and all changes coming within the equivalent scope of the present invention are intended to be included in the scope of the present invention. The present invention is not intended to be limited to the embodiments set forth herein but is to be accorded the widest scope consistent with the principles and novel features disclosed herein. In addition, claims that do not have an explicit citation relationship in the claims may be combined to constitute an embodiment or may be included as a new claim by post-application amendment.

In the emergency treatment device for chest compression and defibrillation of the patient and the method for correcting for loss of the chest compression depth of the patient according to the present invention, it is possible to correct for the loss of the chest compression depth due to the change in the chest shape that occurs in the process of compressing the chest of the patient with the cap provided in the emergency treatment device for the chest compression and defibrillation of the patient, thereby ensuring that the chest of the patient is compressed accurately. Therefore, the present invention has industrial applicability.

In addition, in the emergency treatment device for chest compression and defibrillation of the patient and the method for correcting for loss of the chest compression depth of the patient according to the present invention, it is possible to automate the process of the chest compression and defibrillation of the patient, perform the compression at the existing compression depth when the sum of the set initial chest compression depth and the separation distance is less than the threshold value of a compression depth, and perform the chest compression at the corrected compression depth when the sum exceeds the threshold value, thereby improving the accuracy and stability of the process of the chest compression and defibrillation of the patient and improving a user's convenience during the chest compression and defibrillation of the patient. Therefore, the present invention has industrial applicability.

Moreover, in the emergency treatment device for chest compression and defibrillation of the patient and the method for correcting for loss of the chest compression depth of the patient according to the present invention, it is possible to detect whether there is the change in the chest compression start position periodically, audibly/visually notify a user of the change in the shape in the chest of the patient, and update the chest compression start position to secure the user's convenience. Therefore, the present invention has industrial applicability.

## Claims

1. An emergency treatment device for chest compression and defibrillation of a patient, the emergency treatment device comprising:
a base plate configured to support a back of the patient;
a support having one end and the other end coupled to both edges of the base plate;
a hood which coupled to one side of the support and positioned above a chest of the patient and to which a piston for compressing the chest of the patient is connected; and
a control unit which sets a value of a chest compression start position of the patient to start chest compression of the patient with a lower end of the piston, and then, expands or contracts the piston so that the lower end of the piston moves to the chest compression start position according to the value of the chest compression start position, applies a value of an initial chest compression depth which is a movement length value of the piston from the chest compression start position toward an upper side of the base plate and a threshold value of the chest compression depth to expand or contract the piston so that the chest compression is performed, detects a change in a shape of the chest of the patient during the chest compression to update the chest compression start position of the patient, compares and determines a value obtained by adding a separation distance between a cap fastened to the lower end of the piston and the chest of the patient and the value of the initial chest compression depth, and the threshold value of the chest compression depth, and expands and contracts the piston so that the cap reciprocates according to the value of the initial or updated chest compression depth to perform the chest compression by the cap.

2. The emergency treatment device of claim 1, further comprising:
a first storage unit in which information on the chest compression start position to which the piston returns after completing chest compression and relaxation of the patient is pre-stored; and
a sensor which detects a current position of the piston in real time and generates information on the current position of the piston.

3. The emergency treatment device of claim 2, wherein the first storage unit pre-stores start time information for starting analysis of electrocardiogram of the patient and end time information for ending the analysis of the electrocardiogram of the patient.

4. The emergency treatment device of claim 2, wherein the control unit, before analyzing the electrocardiogram of the patient, moves the lower end of the piston to the chest compression start position using the chest compression start position information, determines whether the current position information of the piston received from the sensor matches the chest compression start position, and
places the lower end of the piston relatively higher than the chest compression start position so that the piston is in a standby state.

5. The emergency treatment device of claim 4, wherein the control unit includes a plurality of buttons for setting a chest compression mode, the chest compression depth, and a chest compression count of the piston, respectively, and
after analyzing the electrocardiogram of the patient, controls the piston so that the piston compresses and relaxes the chest of the patient according to the setting by the button from the chest compression start position.

6. The emergency treatment device of claim 5, wherein the control unit, before compressing and relaxing the chest of the patient according to the setting by the button, maintains a distance between the lower end of the piston and the chest of the patient at a preset distance so that the chest of the patient is compressed when the piston is lowered by the chest compression depth set through the button, thereby optimizing the chest compression start position of the patient.

7. The emergency treatment device of claim 6, wherein the control unit optimizes the chest compression start position of the patient by updating the chest compression start position of the patient at regular intervals.

8. The emergency treatment device of claim 7, wherein the control unit determines whether the current position information of the piston received from the sensor matches the current position of the piston after moving the lower end of the piston to the chest compression start position, and
receives electrical information used from a motor for driving the piston from the sensor and the current position information of the piston and updates the chest compression start position of the patient when a change in the chest compression start position of the patient is detected.

9. The emergency treatment device of claim 8, wherein the sensor includes a first detection sensor which is equipped in a driving circuit of the motor, detects whether a shape of the chest of the patient changes by using a difference in the electrical information when the lower end of the piston comes into contact with a surface of the chest of the patient at regular intervals, and transmits whether the shape of the chest of the patient changes to the control unit.

10. The emergency treatment device of claim 8, wherein the sensor includes a second detection sensor that detects a rotational speed of the motor and a movement distance of the piston to generate movement distance information of the piston, and transmits the piston movement distance information to the control unit.

11. The emergency treatment device of claim 8, further comprising a first notification unit that outputs a first message for notifying that the chest compression start position of the patient has been updated when the chest compression start position of the patient is updated, and a second message for notifying a difference value between the existing chest compression start position of the patient before the update and the updated chest compression start position of the patient.

12. The emergency treatment device of claim 11, wherein the first notification unit outputs a third message when the difference value between the existing chest compression start position of the patient and the updated chest compression start position of the patient is equal to or more than a specific threshold value, a fourth message for requesting a user to select the chest compression depth and the chest compression count of the piston when the chest compression start position of the patient is updated, and a fifth message for notifying the user that the shape of the chest of the patient has changed when the first detection sensor detects the change in the shape of the chest of the patient.

13. The emergency treatment device of claim 12, wherein the control unit updates the chest compression depth and the chest compression count when the chest compression depth and the chest compression count are selected through the plurality of buttons after outputting the fourth message from the first notification unit.

14. The emergency treatment device of claim 13, further comprising a visual display device having an LED implemented as an LED light board or an LED flashing light for visually outputting at least one of the first, second, third, fourth, and fifth messages output from the first notification unit, and a display.

15. The emergency treatment device of claim 1, wherein the hood moves according to sliding movement of the support.

16. The emergency treatment device of claim 1, wherein the base plate includes a band having a pair of cuffs disposed on a side portion to wrap around the upper portion of an arm including an elbow of the patient while the chest of the patient is compressed and fix the arm of the patient.

17. A method for correcting for loss of a chest compression depth performed in an emergency treatment device for chest compression and defibrillation of a patient, the method comprising:
a) setting a value of a chest compression start position of the patient to start chest compression of the patient with a lower end of a piston positioned above a chest of the patient, and then expanding or contracting the piston so that the lower end of the piston moves to the chest compression start position according to the value of the chest compression start position of the patient, by a control unit;
b) applying a value of an initial chest compression depth which is a movement length value of the piston from the chest compression start position of the patient toward an upper side of a base plate supporting a back of the patient and a threshold value of the chest compression depth to expand and contract the piston so that chest compression is performed, and detecting a change in a shape of the chest of the patient during the chest compression to update the chest compression start position of the patient, by the control unit;
c) comparing and determining a value obtained by adding a separation distance between a cap fastened to the lower end of the piston and the chest of the patient and the value of the initial chest compression depth and the threshold value of the chest compression depth, by the control unit; and
d) expanding and contracting the piston so that the cap reciprocates according to the value of the initial or updated chest compression depth to perform the chest compression by the cap.

18. The method of claim 17, wherein in the a), after the control unit receives a position value of the lower end of the piston from a first detection sensor, the control unit adds a value of a pre-input width of a first housing positioned between the lower end of the piston and the chest of the patient to the position value of the lower end of the piston to determine a current position of the piston, determines whether the determined current position of the piston is the same as the chest compression start position, and expands or contracts the piston so that the determined current position of the piston is the same as the value of the chest compression start position.

19. The method of claim 18, wherein the b) includes,
b-1) detecting whether the shape of the chest of the patient changes by using a difference in electrical information by the first detection sensor when the lower end of the piston comes into contact with a chest surface of the patient while the chest compression is performed by the piston,
b-2) notifying a user whether the shape of the chest of the patient changes by a first notification unit when the shape of the chest of the patient changes, and
b-3) updating the chest compression start position of the patient by the control unit when the user inputs a signal to an adaptive length change button for updating the chest compression start position of the patient.

20. The method of claim 19, wherein the d) includes
d-1) calculating an absolute value of a difference between the sum of the value of the separation distance and the value of the initial chest compression depth, and the threshold value of the chest compression depth by the control unit when the sum of the initial chest compression depth value and the separation distance value exceeds the threshold value of the chest compression depth,
d-2) updating the value of the chest compression depth by subtracting the absolute value from the value of the chest compression depth by the control unit,
d-3) controlling the first notification unit by the control unit so that a message according to the updated value of the chest compression depth is output, and
d-4) expanding and contracting, by the control unit, the piston so that the cap reciprocates according to the updated value of the chest compression depth after outputting the message to perform the chest compression by the cap.

21. The method of claim 20, wherein the first notification unit sequentially and repeatedly outputs a first message for notifying that the chest compression start position of the patient has been updated when the chest compression start position of the patient is updated until the user of the emergency treatment device inputs the signal to the control unit, and a second message for notifying a difference value between the existing chest compression start position of the patient before the update and the updated chest compression start position of the patient, and transmits information on the first message and the second message to the control unit in real time.

22. The method of claim 21, wherein the control unit expands or contracts the piston so that the cap reciprocates according to the updated value of the chest compression depth to perform the chest compression by the cap when each of the first and second messages is output once.

23. The method of claim 17, wherein in the d), when the sum of the separation distance between the chest of the patient and the cap and the initial chest compression depth is less than the threshold value of the chest compression depth, the control unit expands and contracts the piston so that the cap reciprocates according to the set chest compression depth value to perform the chest compression by the cap.
